# EUROPEAN PATENT APPLICATION

(11) **EP 2 072 061 A1**
(43) Date of publication of application: **24.06.2009**
(21) Application number: 07024585.7
(22) Date of filing: 19.12.2007
(51) Int. Cl.: A61K 49/00

(54) **Composition and method for generating a metabolic profile using 13C-MR detection**

(71) Applicant: GE Healthcare Limited, Little Chalfont, Buckinghamshire HP7 9NA (GB)
(72) Inventor: Gisselsson, Anna, 200 41 Malmö (SE); Hansson, Georg, 200 41 Malmö (SE); Mansson, Sven, 200 41 Malmö (SE); In't Zandt, René, 200 41 Malmö (SE); Karlsson, Magnus, 200 41 Malmö (SE); Jensen, Pernille R., 200 41 Malmö (SE); Lerche, Mathilde H., 200 41 Malmö (SE)
(74) Representative: Flechsler, Insa

(57) **Abstract**

The invention relates to a method of ¹³C-MR detection using an imaging medium comprising hyperpolarised ¹³C-fumarate and imaging media comprising hyperpolarised ¹³C-fumarate for use in said method.

## Description

The invention relates to a method of ¹³C-MR detection using an imaging medium comprising hyperpolarised ¹³C-fumarate and imaging media comprising hyperpolarised ¹³C-fumarate for use in said method.

Magnetic resonance (MR) imaging (MRI) is a technique that has become particularly attractive to physicians as images of a patients body or parts thereof can be obtained in a non-invasive way and without exposing the patient and the medical personnel to potentially harmful radiation such as X-rays. Because of its high quality images and good spatial and temporal resolution, MRI is a favourable imaging technique for imaging soft tissue and organs.

MRI may be carried out with or without MR contrast agents. However, contrast-enhanced MRI usually enables the detection of much smaller tissue changes which makes it a powerful tool for the detection of early stage tissue changes like for instance small tumours or metastases.

Several types of contrast agents have been used in MRI. Water-soluble paramagnetic metal chelates, for instance gadolinium chelates like Omniscan^{™} (GE Healthcare) are widely used MR contrast agents. Because of their low molecular weight they rapidly distribute into the extracellular space (i.e. the blood and the interstitium) when administered into the vasculature. They are also cleared relatively rapidly from the body.

Blood pool MR contrast agents on the other hand, for instance superparamagnetic iron oxide particles, are retained within the vasculature for a prolonged time. They have proven to be extremely useful to enhance contrast in the liver but also to detect capillary permeability abnormalities, e.g. "leaky" capillary walls in tumours which are a result of tumour angiogenesis.

Despite the undisputed excellent properties of the aforementioned contrast agents their use is not without any risks. Although paramagnetic metal chelates have usually high stability constants, it is possible that toxic metal ions are released in the body after administration. Further, these type of contrast agents show poor specificity.

WO-A-99/35508 discloses a method of MR investigation of a patient using a hyperpolarised solution of a high T₁ agent as MRI contrast agent. The term "hyperpolarisation" means enhancing the nuclear polarisation of NMR active nuclei present in the high T₁ agent, i.e. nuclei with non-zero nuclear spin, preferably ¹³C- or ¹⁵N-nuclei. Upon enhancing the nuclear polarisation of NMR active nuclei, the population difference between excited and ground nuclear spin states of these nuclei is significantly increased and thereby the MR signal intensity is amplified by a factor of hundred and more. When using a hyperpolarised ¹³C- and/or ¹⁵N-enriched high T₁ agent, there will be essentially no interference from background signals as the natural abundance of ¹³C and/or ¹⁵N is negligible and thus the image contrast will be advantageously high. The main difference between conventional MRI contrast agents and these hyperpolarised high T₁ agents is that in the former changes in contrast are caused by affecting the relaxation times of water protons in the body whereas the latter class of agents can be regarded as non-radioactive tracers, as the signal obtained arises solely from the agent.

A variety of possible high T₁ agents for use as MR imaging agents are disclosed in WO-A-99/35508, including non-endogenous and endogenous compounds. As examples of the latter intermediates in normal metabolic cycles are mentioned which are said to be preferred for imaging metabolic activity. By in vivo imaging of metabolic activity, information of the metabolic status of a tissue may be obtained and said information may for instance be used to discriminate between healthy and diseased tissue.

For instance pyruvate is a compound that plays a role in the citric acid cycle and the conversion of hyperpolarised ¹³C-pyruvate to its metabolites hyperpolarised ¹³C-lactate, hyperpolarised ¹³C-bicarbonate and hyperpolarised ¹³C-alanine can be used for *in vivo* MR studying of metabolic processes in the human body.

The metabolic conversion of hyperpolarised ¹³C-pyruvate to its metabolites hyperpolarised ¹³C-lactate, hyperpolarised ¹³C-bicarbonate and hyperpolarised ¹³C-alanine can be used for *in vivo* MR study of metabolic processes in the human body since said conversion has been found to be fast enough to allow signal detection from the parent compound, i.e. hyperpolarised ¹³C₁-pyruvate, and its metabolites. The amount of alanine, bicarbonate and lactate is dependent on the metabolic status of the tissue under investigation. The MR signal intensity of hyperpolarised ¹³C-lactate, hyperpolarised ¹³C-bicarbonate and hyperpolarised ¹³C-alanine is related to the amount of these compounds and the degree of polarisation left at the time of detection, hence by monitoring the conversion of hyperpolarised ¹³C-pyruvate to hyperpolarised ¹³C-lactate, hyperpolarised ¹³C-bicarbonate and hyperpolarised ¹³C-alanine it is possible to study metabolic processes *in vivo* in the human or non-human animal body by using non-invasive MR imaging and/or MR spectroscopy.

The MR signal amplitudes arising from the different pyruvate metabolites vary depending on the tissue type. The unique metabolic peak pattern formed by alanine, lactate, bicarbonate and pyruvate can be used as fingerprint for the metabolic state of the tissue under examination.

Hyperpolarised ¹³C-pyruvate may for instance be used as an MR imaging agent for assessing the viability of myocardial tissue by MR imaging as described in detail in WO-A-2006/054903 and for *in vivo* tumour imaging as described in detail in WO-A-2006/011810.

Tumour tissue is often characterised by an increased perfusion and higher metabolic activity. The process of increasing the vascular bed, angiogenesis, is induced by cells that due to their higher metabolic needs and/or their larger distance from a capillary are not able to get enough substrates that can provide the energy needed to sustain energy homeostasis. It is in this area, where cells have problems in producing enough energy a marked change in metabolic pattern is expected. Tissue with problems sustaining energy homeostasis will alter its energy metabolism which in particular results in an increased lactate production. With the use of hyperpolarised ¹³C-pyruvate as an MR imaging agent, this higher metabolic activity can be seen by an increased production of ¹³C-lactate which can be detected by ¹³C-MR detection.

However, since the production of hyperpolarised ¹³C-pyruvate which is suitable as an *in vivo* imaging agent is not without challenges, there is a need of alternative hyperpolarised imaging agents which can be used to obtain information about metabolic activity, especially in the field of oncology.

We have now found that hyperpolarised ¹³C-fumarate may be used as such an imaging agent.

Fumarate is metabolized to malate by fumarate hydratase or to succinate by succinate dehydrogenase. Both of these metabolic reactions are part of the citric acid cycle and take place in the mitochondrion. However, fumarate is also a by-product of the urea cycle and the formation of fumarate takes place in the cytosol where a cytosolic isoform of fumarate hydratase converts it to malate. Hence by using hyperpolarized ¹³C-fumarate as an imaging agent, both metabolic cycles can be targeted and investigated.

Thus, in a first aspect the invention provides a method of ¹³C-MR detection using an imaging medium comprising hyperpolarised ¹³C-fumarate wherein signals of ¹³C-malate and optionally ¹³C-fumarate and/or ¹³C-succinate are detected.

The term "signals of ¹³C-malate and optionally ¹³C-fumarate and/or ¹³C-succinate are detected" means that in the method of the invention, only the signal of ¹³C-malate is detected or the signals of ¹³C-malate and ¹³C-fumarate are detected or the signals of ¹³C-malate and ¹³C-succinate are detected or the signals of ¹³C-malate and ¹³C-fumarate and ¹³C-succinate are detected.

The term "¹³C-MR detection" denotes ¹³C-MR imaging or ¹³C-MR spectroscopy or combined ¹³C-MR imaging and ¹³C-MR spectroscopy, i.e. ¹³C-MR spectroscopic imaging. The term further denotes ¹³C-MR spectroscopic imaging at various time points.

The term "imaging medium" denotes a liquid composition comprising hyperpolarised ¹³C-fumarate as the MR active agent, i.e. imaging agent.

The imaging medium used in the method of the invention may be used as an imaging medium for *in vivo* ¹³C-MR detection, i.e. in living human or non-human animal beings. Further, the imaging medium used in the method of the invention may be used as imaging medium for *in vitro* ¹³C-MR detection, e.g. of cell cultures, samples like for instance urine, saliva or blood, *ex vivo* tissue, for instance *ex vivo* tissue obtained from a biopsy or isolated organs, all of those derived from a living human or non-human animal body. In a preferred embodiment, the imaging medium used in the method of the invention may be used as an imaging medium for *in vivo* ¹³C-MR detection

The term "¹³C-fumarate" denotes a salt of ¹³C-fumaric acid that is isotopically enriched with ¹³C, i.e. in which the amount of ¹³C isotope is greater than its natural abundance. Unless otherwise specified, the terms "¹³C-fumarate" and "¹³C-fumaric acid" denote a compound which is ¹³C-enriched at one or both of the two carbonyl carbon atoms present in the molecule, i.e. at the C1-position or at the C1- and the C4-position.

The isotopic enrichment of the hyperpolarised ¹³C-fumarate used in the method of the invention is preferably at least 75%, more preferably at least 80% and especially preferably at least 90%, an isotopic enrichment of over 90% being most preferred. Ideally, the enrichment is 100%. ¹³C-fumarate used in the method of the invention may be isotopically enriched at the C1-position (in the following denoted ¹³C₁-fumarate) or at the C1- and C4-position (in the following denoted ¹³C_{1,4}-fumarate). Isotopic enrichment at both the C1- and C4-position is most preferred.

Further, deuterated ¹³C-fumarate may be used in the method of the invention, i.e. one or more hydrogen atoms in ¹³C-fumarate may be exchanged by deuterium atoms. In a preferred embodiment, ¹³C-fummte-d₂ is used in the method of the invention, i.e. ¹³C-fumarate wherein the two hydrogen atoms of the ethenylene group are exchanged by deuterium atoms. In a more preferred embodiment, ¹³C₁₋₄-fumarate-d₂ is used.

The term "¹³C-malate" denotes a salt of ¹³C-malic acid that is isotopically enriched with ¹³C, i.e. in which the amount of ¹³C isotope is greater than its natural abundance. Unless otherwise specified, the term "¹³C-malate" denotes a compound which is ¹³C-enriched at one or both of the two carbonyl carbon atoms present in the molecule, i.e. at the C1-position or at the C1- and the C4-position. If ¹³C₁-fumarate was used in the method of the invention, the signals of ¹³C₁-malate and ¹³C₄-malate are detected. This is due to the break in symmetry of fumarate, when hydrated to malate. If ¹³C_{1,4}-fumarate was used in the method of the invention, the signal of ¹³C_{1,4}-malate is detected, which is twice as high as the malate signal obtained from the conversion of ¹³C₁-fumarate. The situation is illustrated in Scheme 1:

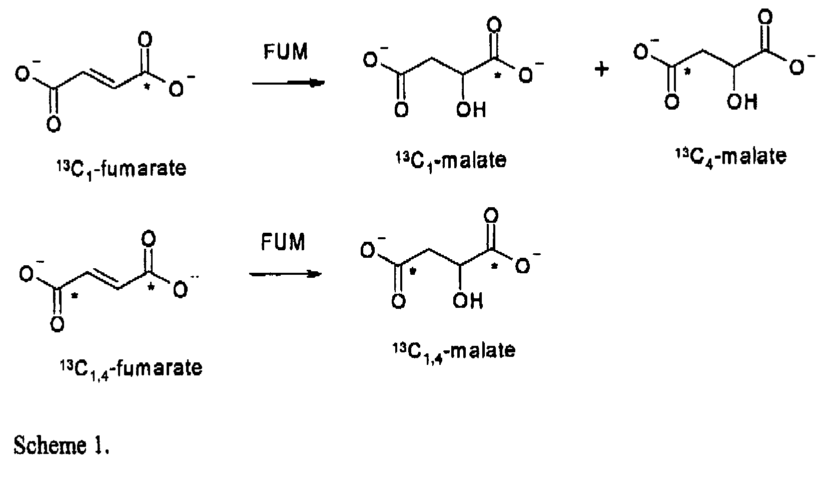

The term "¹³C-succinate" denotes a salt of ¹³C-succiaic acid that is isotopically enriched with ¹³C, i.e. in which the amount of ¹³C isotope is greater than its natural abundance. Unless otherwise specified, the term "¹³C-succinate" denotes a compound which is ¹³C-enriched at one or both of the two carbonyl carbon atoms present in the molecule, i.e. at the C 1-position or at the C1- and the C4-position. The position of the isotopic enrichment in ¹³C-succinate is of course dependent on the position of the isotopic enrichment in its parent compound ¹³C-fumarate. Thus, if ¹³C₁-fumarate was used in the method of the invention, the signal of ¹³C₁-succinate is optionally detected. If ¹³C_{1,4}-fumarate was used in the method of the invention, the signal of ¹³C_{1,4}-suceinate is optionally detected.

The terms "hyperpolarised" and "polarised" are used interchangeably hereinafter and denote a nuclear polarisation level in excess of 0.1%, more preferred in excess of 1% and most preferred in excess of 10%.

The level of polarisation may for instance be determined by solid state ¹³C-NMR measurements in solid hyperpolarised ¹³C-fumarate, e.g. solid hyperpolarised ¹³C-fumarate obtained by dynamic nuclear polarisation (DNP) of ¹³C-fumarate. The solid state ¹³C-NMR measurement preferably consists of a simple pulse-acquire NMR sequence using a low flip angle. The signal intensity of the hyperpolarised ¹³C-fumarate in the NMR spectrum is compared with signal intensity of ¹³C-fumarate in a NMR spectrum acquired before the polarisation process. The level of polarisation is then calculated from the ratio of the signal intensities of before and after polarisation.

In a similar way, the level of polarisation for dissolved hyperpolarised ¹³C-fumarate may be determined by liquid state NMR measurements. Again the signal intensity of the dissolved hyperpolarised ¹³C-fumarate is compared with the signal intensity of the dissolved ¹³C-fumarate before polarisation. The level of polarisation is then calculated from the ratio of the signal intensities of ¹³C-fumarate before and after polarisation.

Hyperpolarisation of NMR active ¹³C-nuclei may be achieved by different methods which are for instance described in described in WO-A-98/30918, WO-A-99/24080 and WO-A-99/35508, and which all are incorporated herein by reference and hyperpolarisation methods known in the art are polarisation transfer from a noble gas, "brute force", spin refrigeration, the parahydrogen method and dynamic nuclear polarisation (DNP).

To obtain hyperpolarised ¹³C-fumarate, it is preferred to polarise ¹³C-fumarate directly. Also ¹³C-fumaric acid may be polarised and the polarised ¹³C-fumaric acid is subsequently converted to polarised ¹³C-fumarate, e.g. by neutralisation with a base. Suitable ¹³C-fumarates which can be used in the polarisation procedure can be prepared from commercially available ¹³C-fumaric acid.

One way for obtaining hyperpolarised ¹³C-fumarate is the polarisation transfer from a hyperpolarised noble gas which is described in WO-A-98/30918. Noble gases having non-zero nuclear spin can be hyperpolarised by the use of circularly polarised light. A hyperpolarised noble gas, preferably He or Xe, or a mixture of such gases, may be used to effect hyperpolarisation of ¹³C-nuclei. The hyperpolarised gas may be in the gas phase, it may be dissolved in a liquid/solvent, or the hyperpolarised gas itself may serve as a solvent Alternatively, the gas may be condensed onto a cooled solid surface and used in this form, or allowed to sublime. Intimate mixing of the hyperpolarised gas with ¹³C-fumarate or ¹³C-fumaric acid is preferred.

Another way for obtaining hyperpolarised ¹³C-fumarate is that polarisation is imparted to ¹³C-nuclei by thermodynamic equilibration at a very low temperature and high field. Hyperpolarisation compared to the operating field and temperature of the NMR spectrometer is effected by use of a very high field and very low temperature (brute force). The magnetic field strength used should be as high as possible, suitably higher than 1 T, preferably higher than 5 T, more preferably 15 T or more and especially preferably 20 T or more. The temperature should be very low, e.g. 4.2 K or less, preferably 1.5 K or less, more preferably 1.0 K or less, especially preferably 100 mK or less.

Another way for obtaining hyperpolarised ¹³C-fumarate is the spin refrigeration method. This method covers spin polarisation of a solid compound or system by spin refrigeration polarisation. The system is doped with or intimately mixed with suitable crystalline paramagnetic materials such as Ni²⁺, lanthanide or actinide ions with a symmetry axis of order three or more. The instrumentation is simpler than required for DNP with no need for a uniform magnetic field since no resonance excitation field is applied. The process is carried out by physically rotating the sample around an axis perpendicular to the direction of the magnetic field. The prerequisite for this method is that the paramagnetic species has a highly anisotropic g-factor. As a result of the sample rotation, the electron paramagnetic resonance will be brought in contact with the nuclear spins, leading to a decrease in the nuclear spin temperature. Sample rotation is carried out until the nuclear spin polarisation has reached a new equilibrium.

In a preferred embodiment, DNP (dynamic nuclear polarisation) is used to obtain hyperpolarised ¹³C-fumarate. In DNP, polarisation of MR active nuclei in a compound to be polarised is affected by a polarisation agent or so-called DNP agent, a compound comprising unpaired electrons. During the DNP process, energy, normally in the form of microwave radiation, is provided, which will initially excite the DNP agent. Upon decay to the ground state, there is a transfer of polarisation from the unpaired electron of the DNP agent to the NMR active nuclei of the compound to be polarised, e.g. to the ¹³C nuclei in ¹³C-fumarate or ¹³C-fumaric acid. Grenerally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. The DNP technique is for example further described in WO-A-98/58272 and in WO-A-01/96895, both of which are included by reference herein.

To polarise a chemical entity, i.e. compound, by the DNP method, a composition of the compound to be polarised and a DNP agent is prepared which is then optionally frozen and inserted into a DNP polariser (where it will freeze if it has not been frozen before) for polarisation. After the polarisation, the frozen solid hyperpolarised composition is rapidly transferred into the liquid state either by melting it or by dissolving it in a suitable dissolution medium. Dissolution is preferred and the dissolution process of a frozen hyperpolarised composition and suitable devices therefore are described in detail in WO-A-02/37132. The melting process and suitable devices for the melting are for instance described in WO-A-02/3b005.

In order to obtain a high polarisation level in the compound to be polarised said compound and the DNP agent need to be in intimate contact during the DNP process. This is not the case if the composition crystallizes upon being frozen or cooled. To avoid crystallization, either glass formers need to be present in the composition or compounds need to be chosen for polarisation which do not crystallize upon being frozen but rather form a glass.

In one embodiment, ¹³C-fumaric acid, preferably ¹³C_{1,4}-fumaric acid and more preferably ¹³C_{1,4}-fumaric acid-d₂ is used as a starting material to obtain hyperpolarised ¹³C-fumarate by the DNP method.

In another embodiment, ¹³C-fumarate, preferably ¹³C_{1,4}-fumarate and more preferably ¹³C_{1,4}-fumarate-d₂ is used as a starting material to obtain hyperpolarised ¹³C-fumarate by the DNP method.

A suitable ¹³C-fumarate is for instance sodium ¹³C-fumarate. Alternatively, ¹³C-fumarates which comprise an inorganic cation from the group consisting of NH₄⁺, K⁺, Rb⁺, Cs⁺, Ca²⁺, Sr²⁺ and Ba²⁺. The latter salts are described in detail in WO-A-2007/111515 which is incorporated by reference herein. Preferably, ¹³C-fumarates of an organic amine or amino compound, more preferably TRIS-¹³C-fumarate or meglumine-¹³C-fumarate, may be used. These salts are in detail described in WO-A-2007/069909, which is incorporated by reference herein.

In a most preferred embodiment, TRIS-¹³C₁-fumarate and more preferably TRIS-¹³C_{1,4}-fumarate is used as a starting material to obtain hyperpolarised ¹³C-fumarate by the DNP method.

The term "TRIS" denotes 2-amino-2-hydroxymethyl-1,3-propanediol and the term "TRIS-¹³C-fumarate" denotes a salt which contains ¹³C-fumarate as anion and a TRIS cation, i.e. TRIS ammonium (2-hydroxymethyl-1,3-propanedioyl ammonium). The term TRIS-¹³C-fumarate denotes both the mono- and di-TRIS salt of ¹³C-fumaric acid. In one embodiment, the mono-TRIS salt of ¹³C-fumaric acid is used as a starting material to obtain hyperpolarised ¹³C-fumarate by converting the carboxy group in said hyperpolarised mono-TRIS salt of ¹³C-fumaric acid into a salt by using a base. Said base may for instance be a present in the dissolution medium used to dissolve the solid hyperpolarised mono-TRIS salt of ¹³C-fumaric acid after DNP polarisation. In another embodiment, the di-TRIS salt of ¹³C-fumaric acid is used as a starting material to obtain hyperpolarised ¹³C-fumarate.

For the hyperpolarisation of ¹³C-fumarate by the DNP method, a composition is prepared which comprises ¹³C-fumarate or ¹³C-fumaric acid and a DNP agent.

The DNP agent plays a decisive role in the DNP process as its choice has a major impact on the level of polarisation that can be achieved in ¹³C-fumarate. A variety of DNP agents - in WO-A-99/35508 denoted "QMRI contrast agents" - is known. The use of oxygen-based, sulphur-based or carbon-based stable trityl radicals as described in WO-A-99/35508, WO-A-88/10419, WO-A-90/00904, WO-A-91/12024, WO-A-93/02711 or WO-A-96/39367 has resulted in high levels of polarisation in a variety of different samples.

In a preferred embodiment, the hyperpolarised ¹³C-fumarate used in the method of the invention is obtained by DNP and the DNP agent used is a trityl radical. As briefly mentioned above, the large electron spin polarisation of the DNP agent, i.e. trityl radical is converted to nuclear spin polarisation of ¹³C nuclei in ¹³C-fumarate or ¹³C-fumaric acid via microwave irradiation close to the electron Larmor frequency. The microwaves stimulate communication between electron and nuclear spin systems via e-e and e-n transitions. For effective DNP, i.e. to achieve a high level of polarisation in ¹³C-fumarate or ¹³C-fumaric acid, the trityl radical has to be stable and soluble in these compounds to achieve said intimate contact between ¹³C-fumarate or ¹³C-fumaric acid and the trityl radical which is necessary for the aforementioned communication between electron and nuclear spin systems.
In a preferred embodiment, the trityl radical is a radical of the formula (1) 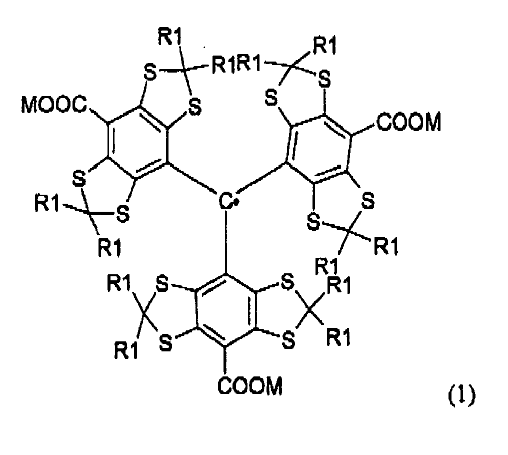 wherein
- M: represents hydrogen or one equivalent of a cation; and
- R1: which is the same or different represents a straight chain or branched C₁-C₆-alkyl group optionally substituted by one or more hydroxyl groups or a group -(CH₂)ₙ-X-R₂,
wherein n is 1, 2 or 3;
X is O or S; and
R2 is a straight chain or branched C₁-C₄-awyl group, optionally substituted by one or more hydroxyl groups.

In a preferred embodiment, M represents hydrogen or one equivalent of a physiologically tolerable cation. The term "physiologically tolerable cation" denotes a cation that is tolerated by the human or non-human animal living body. Preferably, M represents hydrogen or an alkali cation, an ammonium ion or an organic amine ion, for instance meglumine. Most preferably, M represents hydrogen or sodium.

If ¹³C-fumarate is used as a starting material to obtain hyperpolarised ¹³C-fumarate by the DNP method, R1 is preferably the same, more preferably a straight chain or branched C₁-C₄-alkyl group, most preferably methyl, ethyl or isopropyl; or R1 is preferably the same, more preferably a straight chain or branched C₁-C₄-alkyl group which is substituted by one hydroxyl group, most preferably -CH₂-CH₂-OH; or R1 is preferably the same and represents -CH₂-OC₂H₄OH.

If ¹³C-fumaric acid is used as a starting material to obtain hyperpolarised ¹³C-fumarate by the DNP method, R1 is the same or different, preferably the same and preferably represents -CH₂-OCH₃, -CH₂-OC₂H₅, -CH₂-CH₂-OCH₃, -CH₂-SCH₃, - CH₂-SC₂H₅ or -CH₂-CH₂-SCH₃, most preferably -CH₂-CH₂-OCH₃.

The aforementioned trityl radicals of formula (1) may be synthesized as described in detail in WO-A-88/10419, WO-A-90/00904, WO-A-91/12024, WO-A-93/02711, WO-A-96/39367, WO-A-97/09633, WO-A-98/39277 and WO-A-2006/011811.

Generally, for the DNP process, a solution of the starting material, i.e. ¹³C-fumaric acid or ¹³C-fumarate (in the following denoted "sample") and the DNP agent, preferably a trityl radical, more preferably a trityl radical of formula (1) is prepared. A solvent or a solvent mixture needs to be used to promote dissolution of the DNP agent and the sample. If the hyperpolarised ¹³C-fumarate is intended to be used as an imaging agent for in vivo ¹³C-MR detection, it is preferred to keep the amount of solvent to a minimum. To be used as an *in vivo* imaging agent, the polarised ¹³C-fumarate is usually administered in relatively high concentrations, i.e. a highly concentrated sample is preferably used in the DNP process and hence the amount of solvent is preferably kept to a minimum. In this context, it is also important to mention that the mass of the composition containing the sample, i.e. DNP agent, sample and if necessary solvent, is kept as small as possible. A high mass will have a negative impact on the efficiency of the dissolution process, if dissolution is used to convert the solid composition containing the hyperpolarised ¹³C-fumaric acid or ¹³C-fumarate after the DNP process into the liquid state, e.g. for using it as an imaging agent for ¹³C-MR detection. This is due to the fact that for a given volume of dissolution medium in the dissolution process, the mass of the composition to dissolution medium ratio decreases, when the mass of the composition increases. Further, using certain solvents may require their removal before the hyperpolarised ¹³C-fumarate used as an MR imaging agent is administered to a human or non-human animal being since they might not be physiologically tolerable.

If ¹³C-fumaric acid is used as a starting material to obtain hyperpolarised ¹³C-fumarate via DNP, a solution of the DNP agent, preferably a trityl radical and more preferably a trityl radical of formula (1) and ¹³C-fumaric acid in a solvent or solvent mixture is prepared. ¹³C-fumaric acid is a solid at room temperature and DMSO is preferably used a solvent since ¹³C-fumaric acid is little soluble in water. The mixture of ¹³C-fumaric acid and DMSO may be gently heated and optionally sonicated to achieve dissolution. Since ¹³C-fumaric acid in DMSO does not crystallize upon freezing, the addition of any glass former like for instance glycerol is not mandatory but optional.

If a ¹³C-fumarate of an organic amine or amino compound like TRIS-¹³C-fumarate is used as the starting material, it is preferably generated *in situ,* i.e. by dissolving ¹³C-fumaric acid in a solvent or solvent mixture, preferably water and adding said organic amine or amino compound. The resulting salt can be isolated or used further without isolating it. The advantage of isolating the salt is that it can be characterized and one knows exactly if a base needs to be added to the dissolution medium and in which amounts. This makes it easy to control the pH of the final imaging medium. A glass former is optionally added to the dissolved ¹³C-fumarate of an organic amine or amino compound. The DNP agent, preferably a trityl radical and more preferably a trityl radical of formula (1) is then preferably added to the solution of ¹³C-fumarate. Again intimate mixing of the compounds can be promoted by several means known in the art, such as stirring, vortexing or sonication and/or gentle heating.

If the hyperpolafised ¹³C-fumarate used in the method of the invention is obtained by DNP, the composition to be polarised comprising ¹³C-fumaric acid or ¹³C-fumarate and a DNP agent may further comprise a paramagnetic metal ion. It has been found that the presence of paramagnetic metal ions may result in increased polarisation levels in the compound to be polarised by DNP as described in detail in WO-A2-2007/064226 which is incorporated herein by reference.

The term "paramagnetic metal ion" denotes paramagnetic metal ions in the form of their salts or in chelated form, i.e. paramagnetic chelates. The latter are chemical entities comprising a chelator and a paramagnetic metal ion, wherein said paramagnetic metal ion and said chelator form a complex, i.e. a paramagnetic chelate.

In a preferred embodiment, the paramagnetic metal ion is a salt or paramagnetic chelate comprising Gd³⁺, preferably a paramagnetic chelate comprising Gd³⁺. In a more preferred embodiment, said paramagnetic metal ion is soluble and stable in the composition to be polarised.

As with the DNP agent described before, the ¹³C-fumaric acid or ¹³C-fumarate to be polarised must be in intimate contact with the paramagnetic metal ion as well. The composition used for DNP comprising ¹³C-fumaric acid or ¹³C-fumarate, a DNP agent and a paramagnetic metal ion may be obtained in several ways.

In a first embodiment the ¹³C-fumarate is dissolved in a suitable solvent to obtain a solution, alternatively the ¹³C-fumarate is *in situ* generated by mixing ¹³C-fumaric acid with a base like an organic amine or amino compound and a solvent, preferably water. In an alternative first embodiment, ¹³C-fumaric acid is dissolved in a suitable solvent, preferably DMSO to obtain a solution. To these solutions of ¹³C-fumarate or ¹³C-fumaric acid the DNP agent is added and dissolved. The DNP agent, preferably a trityl radical, might be added as a solid or in solution, e.g. dissolved in water or DMSO. In a subsequent step, the paramagnetic metal ion is added. The paramagnetic metal ion might be added as a solid or in solution, e.g. dissolved in water or DMSO. In another embodiment, the DNP agent and the paramagnetic metal ion are dissolved in suitable solvents or a suitable solvent, e.g. water or DMSO and to this solution is added ¹³C-fumaric acid or ¹³C-fumarate. In yet another embodiment, the DNP agent (or the paramagnetic metal ion) is dissolved in a suitable solvent and added to optionally dissolved ¹³C-fumaric acid or ¹³C-fumarate In a subsequent step the paramagnetic metal ion (or the DNP agent) is added to this solution, either as a solid or in solution. Preferably, the amount of solvent to dissolve the paramagnetic metal ion (or the DNP agent) is kept to a minimum. Again intimate mixing of the compounds can be promoted by several means known in the art, such as stirring, vortexing or sonication and/or gentle heating.

If a trityl radical is used as DNP agent, a suitable concentration of such a trityl radical is 1 to 25 mM, preferably 2 to 20 mM, more preferably 10 to 15 mM in the composition used for DNP. If a paramagnetic metal ion is added to the composition, a suitable concentration of such a paramagnetic metal ion is 0.1 to 6 mM (metal ion) in the composition, and a concentration of 0.3 to 4 mM is preferred.

After having prepared a composition comprising ¹³C-fumaric acid or ¹³C-fumarate, the DNP agent and optionally a paramagnetic metal ion said composition is frozen by methods known in the art, e.g. by freezing it in a freezer, in liquid nitrogen or by simply placing it in the DNP polariser, where liquid helium will freeze it. In a preferred embodiment, the composition is frozen as "beads" before it is inserted into to polariser. Such beads may be obtained by adding the composition drop wise to liquid nitrogen. A more efficient dissolution of such beads has been observed, which is especially relevant if larger amounts of ¹³C-fumaric acid or ¹³C-fumarate are polarised, for instance when it is intended to use the polarised ¹³C-fumarate in an *in vivo* ¹³C-MR detection method.

If a paramagnetic metal ion is present in the composition said composition may be degassed before freezing, e.g. by bubbling helium gas through the composition (e.g. for a time period of 2 - 15 min) but degassing can be effected by other known common methods.

The DNP technique is for instance described in WO-A-98/58272 and in WO-A-01/96895, both of which are included by reference herein. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. In a preferred embodiment, the DNP process is carried out in liquid helium and a magnetic field of about 1 T or above. Suitable polarisation units are for instance described in WO-A-02/37132. In a preferred embodiment, the polarisation unit comprises a cryostat and polarising means, e.g. a microwave chamber connected by a wave guide to a microwave source in a central bore surrounded by magnetic field producing means such as a superconducting magnet. The bore extends vertically down to at least the level of a region P near the superconducting magnet where the magnetic field strength is sufficiently high, e.g. between 1 and 25 T, for polarisation of the sample nuclei to take place. The bore for the probe (i.e. the frozen composition to be polarised) is preferably sealable and can be evacuated to low pressures, e.g. pressures in the order of 1 mbar or less. A probe introducing means such as a removable transporting tube can be contained inside the bore and this tube can be inserted from the top of the bore down to a position inside the microwave chamber in region P. Region P is cooled by liquid helium to a temperature low enough to for polarisation to take place, preferably temperatures of the order of 0.1 to 100 K, more preferably 0.5 to 10 K, most preferably 1 to 5 K. The probe introducing means is preferably sealable at its upper end in any suitable way to retain the partial vacuum in the bore. A probe-retaining container, such as a probe-retaining cup, can be removably fitted inside the lower end of the probe introducing means. The probe-retaining container is preferably made of a light-weight material with a low specific heat capacity and good cryogenic properties such, e.g. KelF (polychlorotrifluoroethylene) or PEEK (polyetheretherketone) and it may be designed in such a way that it can hold more than one probe.

The probe is inserted into the probe-retaining container, submerged in the liquid helium and irradiated with microwaves, preferably at a frequency of about 94 GHz at 200 mW. The level of polarisation may be monitored by for instance acquiring solid state ¹³C-NMR signals of the probe during microwave irradiation. Generally, a saturation curve is obtained in a graph showing NMR signal vs. time. Hence it is possible to determine when the optimal and/or sufficient polarisation level is reached. A solid state ¹³C-NMR measurement suitably consists of a simple pulse-acquire NMR sequence using a low flip angle. The signal intensity of the dynamic nuclear polarised nuclei, i.e. ¹³C nuclei in ¹³C-fumaric acid or ¹³C-fumarate is compared with the signal intensity of the ¹³C nuclei in ¹³C-fumaric acid or ¹³C-fumarate before DNP. The polarisation is then calculated from the ratio of the signal intensities before and after DNP.

After the DNP process, the solid composition comprising the hyperpolarised ¹³C-fumaric acid or ¹³C-fumarate is transferred from a solid state to a liquid state, i.e. liquefied. This can be done by dissolution in an appropriate solvent or solvent mixture (dissolution medium) or by melting the solid composition. Dissolution is preferred and the dissolution process and suitable devices therefore are described in detail in WO-A-02/37132. The melting process and suitable devices for the melting are for instance described in WO-A-02/36005. Briefly, a dissolution unit/melting unit is used which is either physically separated from the polariser or is a part of an apparatus that contains the polariser and the dissolution unit/melting unit. In a preferred embodiment, dissolution/melting is carried out at an elevated magnetic field, e.g. inside the polariser, to improve the relaxation and retain a maximum of the hyperpolarisation. Field nodes should be avoided and low field may lead to enhanced relaxation despite the above measures.

If ¹³C-fumarate has been used as the starting material for the dynamic nuclear polarisation and if the solid composition comprising the hyperpolarised ¹³C-fumarate is liquefied by dissolution, an aqueous carrier, preferably a physiologically tolerable and pharmaceutically accepted aqueous carrier like water, a buffer solution or saline is suitably used as a solvent, especially preferably if the hyperpolarised ¹³C-fumarate is intended for use in an imaging medium for in *vivo* ¹³C-MR detection. The aqueous carrier may contain a base to adjust the pH of the final solution in such a way that it is suitable for *in vivo* administration. Suitable pH ranges from 6.8 to 7.8. For *in vitro* applications also non aqueous solvents or solvent mixtures may be used as or in the dissolution medium, for instance DMSO or methanol or mixtures comprising an aqueous carrier and a non aqueous solvent, for instance mixtures of DMSO and water or methanol and water. In another preferred embodiment, the aqueous carrier or the non aqueous solvents or solvent mixtures may further comprise one or more compounds which are able to bind or complex free paramagnetic ions, e.g. chelating agents like DTPA or EDTA.

If ¹³C-fumaric acid or a mono-salt of ¹³C-fumaric acid, e.g. a mono-TRIS salt of ¹³C-fumaric acid has been used as the starting material for the dynamic nuclear polarisation, the hyperpolarised ¹³C-fumaric acid or mono-salt of ¹³C-fumaric acid obtained has to be converted to ¹³C-fumarate. If the solid composition comprising the hyperpolarised ¹³C-fumaric acid or mono-salt of ¹³C-fumaric acid is liquefied by dissolution, the dissolution medium is preferably an aqueous carrier, e.g. water or a buffer solution, preferably a physiologically tolerable buffer solution or it comprises an aqueous carrier, e.g. water or a buffer solution, preferably a physiologically tolerable buffer solution. The terms "buffer solution" and "buffer" are hereinafter used interchangeably. In the context of this application "buffer" denotes one or more buffers, i.e. also mixtures of buffers.

Preferred buffers are physiologically tolerable buffers, more preferably buffers which buffer in the range of about pH 6 to 8 like for instance phosphate buffer (KH₂PO₄Na₂HPO₄), ACES, PIPES, imidazole/HCl, BES, MOPS, HEPES, TES, TRIS, HEPPS or TRICIN.

To convert hyperpolarised ¹³C-fumaric acid or hyperpolarised mono-salt of ¹³C-fumaric acid into hyperpolarised ¹³C-fumarate, the hyperpolarised ¹³C-fumaric acid or mono-salt of ¹³C-fumaric acid is generally reacted with a base. In one embodiment, ¹³C-fumaric acid or mono-salt of ¹³C-fumaric acid is reacted with a base to convert it to ¹³C-fumarate and subsequently an aqueous carrier is added. In another preferred embodiment the aqueous carrier and the base are combined in one solution and this solution is added to ¹³C-fumaric acid or mono-salt of ¹³C-fumaric acid, dissolving it and converting it into ¹³C-fumarate at the same time. In a preferred embodiment, the base is an aqueous solution of NaOH, Na₂CO₃ or NaHCO₃, most preferred the base is NaOH.

In another preferred embodiment, the aqueous carrier buffer or - where applicable - the combined aqueous carrier/base solution further comprises one or more compounds which are able to bind or complex free paramagnetic ions, e.g. chelating agents like DTPA or EDTA.

If hyperpolarisation is carried out by the DNP method, the DNP agent, preferably a trityl radical and the optional paramagnetic metal ion may be removed from the liquid containing the hyperpolarised ¹³C-fumarate. Removal of these compounds is preferred if the hyperpolarised ¹³C-fumarate is intended for use in an imaging medium for *in vivo* use. If ¹³C-fumaric acid or mono-salt of ¹³C-fumaric acid was as a starting material for DNP, it is preferred to first convert the hyperpolarised ¹³C-fumaric acid or mono-salt of ¹³C-fumaric acid into ¹³C-fumarate and remove the DNP agent and the optional paramagnetic metal ion after said conversion has taken place.

Methods which are useful to remove the trityl radical and the paramagnetic metal ion are known in the art and described in detail in WO-A2-2007/064226 and WO-Al-2006/011809.

In a preferred embodiment the hyperpolarised ¹³C-fumarate used in the method of the invention is obtained by dynamic nuclear polarisation of a composition that comprises TRIS-¹³C-fumarate, more preferably TRIS-¹³C_{1,4}-fumarate and most preferably TRIS-¹³C_{1,4}-fumarate-d₂, water, a trityl radical of formula (1) and optionally a paramagnetic chelate comprising Gd³⁺. Optionally, a glass former like glycerol is added.

In another preferred embodiment, the hyperpolarised ¹³C-fumarate used in the method of the invention is obtained by dynamic nuclear polarisation of a composition that comprises ¹³C-fumaric acid, more preferably ¹³C_{1,4}-fumaric acid and most preferably ¹³C_{1,4}-fumaric acid-d₂, DMSO, a trityl radical of formula (1) and optionally a paramagnetic chelate comprising Gd³⁺. Optionally, a glass former like glycerol is added.

The imaging medium according to the method of the invention may be used as imaging medium for *in vitro* ¹³C-MR detection, e.g. ¹³C-MR detection of cell cultures, samples, *ex vivo* tissue or isolated organs derived from the human or non-human animal body. For this purpose, the imaging medium is provided as a composition that is suitable for being added to, for instance, cell cultures, samples like urine, blood or saliva, *ex vivo* tissues like biopsy tissues or isolated organs. Such an imaging medium preferably comprises in addition to the imaging agent, i.e. hyperpolarised ¹³C-fumarate, a solvent which is compatible with and used for *in vitro* cell or tissue assays, for instance an aqueous carrier, DMSO or methanol or solvent mixtures comprising an aqueous carrier and a non aqueous solvent, for instance mixtures of DMSO and water or a buffer solution or methanol and water or a buffer solution. As it is apparent for the skilled person, pharmaceutically acceptable carriers, excipients and formulation aids may be present in such an imaging medium but are not required for such a purpose.

Further, the imaging medium according to the method of the invention may be used as imaging medium for *in vivo* ¹³C-MR detection, i.e. ¹³C-MR detection carried out on living human or non-human animal beings. For this purpose, the imaging medium needs to be suitable for administration to a living human or non-human animal body. Hence such an imaging medium preferably comprises in addition to the imaging agent, i.e. hyperpolarised ¹³C-fumarate, an aqueous carrier, preferably a physiologically tolerable and pharmaceutically accepted aqueous carrier like water, a buffer solution or saline. It may also comprise DMSO which is a solvent that is used in medicinal applications. Such an imaging medium may further comprise conventional pharmaceutical or veterinary carriers or excipients, e.g. formulation aids such as stabilizers, osmolality adjusting agents, solubilising agents and the like which are conventional for diagnostic compositions in human or veterinary medicine.

If the imaging medium used in the method of the invention is used for *in vivo* ¹³C-MR detection, i.e. in a living human or non-human animal body, said imaging medium is preferably administered to said body parenterally, preferably intravenously. Generally, the body under examination is positioned in an MR magnet. Dedicated ¹³C-MR RF-coils are positioned to cover the area of interest. Exact dosage and concentration of the imaging medium will depend upon a range of factors such as toxicity and the administration route. Generally, the imaging medium is administered in a concentration of up to 1 mmol fumarate per kg bodyweight, preferably 0.01 to 0.5 mmol/kg, more preferably 0.1 to 0.3 mmol/kg. At less than 400 s after the administration, preferably less than 120 s, more preferably less than 60 s after the administration, especially preferably 20 to 50 s an MR imaging sequence is applied that encodes the volume of interest in a combined frequency and spatial selective way. The exact time of applying an MR sequence is highly dependent on the volume of interest.

In the method according to the invention, signal of ¹³C-malate and optionally ¹³C-fumarate and/or or ¹³C-succinate. In a preferred embodiment, signals of ¹³C-malate and ¹³C-fumarate are detected.

The metabolic conversion of fumarate to malate and succinate is shown in Scheme 2 for ¹³C_{1,4}-fumarate; * denotes the ¹³C-label: ¹³C-fumarate is hydrated by fumarate hydratase (FUM, EC 4.2.1.2) to form ¹³C-malate and reduced by succinate dehydrogenase (SDH, EC 1.3.5.1) to form ¹³C-succinate.

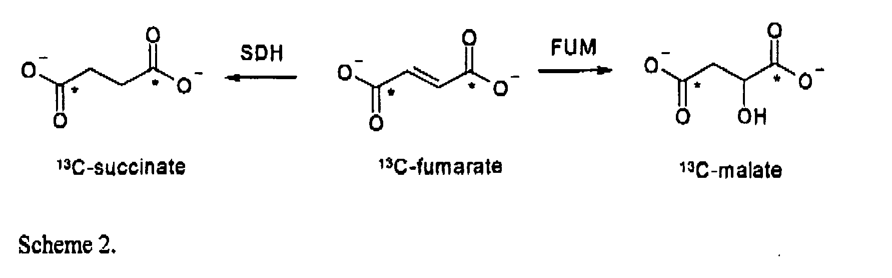

The term "signal" in the context of the invention refers to the MR signal amplitude or integral or peak area to noise of peaks in a ¹³C-MR spectrum which represent ¹³C-malate and optionally ¹³C-fumarate and/or ¹³C-succinate. In a preferred embodiment, the signal is the peak area.

In a preferred embodiment of the method of the invention, the above-mentioned signals of ¹³C-malate and optionally ¹³C-fumarate and/or ¹³C-succinate are used to generate a metabolic profile of a living human or non-human animal being. Said metabolic profile may be derived from the whole body, e.g. obtained by whole body *in vivo* ¹³C-MR detection. Alternatively, said metabolic profile is generated from a region or volume of interest, i.e. a certain tissue, organ or part of said human or non-human animal body.

In another preferred embodiment of the method of the invention, the above-mentioned signals of ¹³C-malate and optionally ¹³C-fumarate and/or ¹³C-succinate are used to generate a metabolic profile of cells in a cell culture, of samples like urine, blood or saliva, of *ex vivo* tissue like biopsy tissue or of an isolated organ derived from a human or non-human animal being. Said metabolic profile is then generated by *in vitro* ¹³C-MR detection.

Thus in a preferred embodiment it is provided a method of ¹³C-MR detection using an imaging medium comprising hyperpolarised ¹³C-fumarate and detecting signals of ¹³C-malate and optionally ¹³C-fumarate and/or ¹³C-succinate, wherein said signals are used to generate a metabolic profile.

In a preferred embodiment, the signals of ¹³C-malate and ¹³C-fumarate are used to generate said metabolic profile.

In one embodiment, the spectral signal intensities of ¹³C-malate and optionally ¹³C-fumarate and/or ¹³C-succinate are used to generate the metabolic profile. In another embodiment, the spectral signal integrals of ¹³C-malate and optionally ¹³C-fumarate and/or ¹³C-succinate are used to generate the metabolic profile. In another embodiment, signal intensities from separate images of ¹³C-malate and optionally ¹³C-fumarate and/or ¹³C-succinate are used to generate the metabolic profile. In yet another embodiment, the signal intensities of ¹³C-malate and optionally ¹³C-fumarate and/or ¹³C-succinate are obtained at two or more time points to calculate the rate of change of ¹³C-malate and optionally ¹³C-fumarate and/or ¹³C-succinate.

In another embodiment the metabolic profile includes or is generated using processed signal data of ¹³C-malate and optionally ¹³C-fumarate and/or ¹³C-succinate, e.g. ratios of signals, corrected signals, or dynamic or metabolic rate constant information deduced from the signal pattern of multiple MR detections, i.e. spectra or images. Thus, in a preferred embodiment a corrected ¹³C-malate signal, i.e. ¹³C-malate to ¹³C-fumarate signal or optionally a corrected ¹³C-succinate signal, i.e. ¹³C-succinate to ¹³C-fumarate signal is included into or used to generate the metabolic profile. In a further preferred embodiment, a corrected ¹³C-malate to total ¹³C-carbon signal or optionally a corrected ¹³C-succinate to total ¹³C-carbon signal is included into or used to generate the metabolic profile with total ¹³C-carbon signal being the sum of the signals of ¹³C-malate and optionally ¹³C-fumarate and/or ¹³C-succinate. In a more preferred embodiment, the ratio of ¹³C-malate to ¹³C-fumarate and optionally the ratio of ¹³C-succinate to ¹³C-fumarate is included into or used to generate the metabolic profile.

The metabolic profile generated in the preferred embodiment of the method according to the invention provides information about the metabolic activity of the body, part of the body, cells, tissue, body sample etc under examination and said information may be used in a subsequent step for, e.g. identifying diseases.

Such a disease is preferably cancer since tumour tissue is usually characterized by a higher metabolic activity than healthy tissue. Such a higher metabolic activity would be apparent from comparing the metabolic profile of a tumour or of an *ex vivo* sample of a tumour with the metabolic profile of healthy tissue (e.g. surrounding tissue or healthy *ex vivo* tissue) and may manifest itself in the metabolic profile by high signals of ¹³C-malate and optionally high signal of ¹³C-succinate or by a high corrected ¹³C-malate or ¹³C-succinate signal or a high ratio of ¹³C-malate to ¹³C-fumarate or to ¹³C-succinate to ¹³C-fumarate or total carbon or a high metabolic rate of ¹³C-malate or ¹³C-succinate build-up.

The term "high" is a relative term and it has to be understood that the "high signal, ratio, metabolic rate" etc. which is seen in a metabolic profile of a diseased tissue as described above is increased compared to the signal, ratio, metabolic rate etc. which is seen in a metabolic profile of a healthy tissue.

Another disease may be ischemia, e.g. ischemia in the heart, since ischemic myocardial tissue is characterized by a higher malate concentration than healthy myocardial tissue. Again such a changed metabolic activity would be apparent from comparing the metabolic profile of ischemic tissue (e.g. ischemic myocardial tissue) with the metabolic profile of healthy tissue (e.g. healthy myocardial tissue) in a way as described in the previous paragraphs.

Yet another disease may be liver related diseases, such as liver fibrosis and liver cirrhosis. In these diseases a higher malate concentration may be characteristic and metabolic profiles can be compared as described above.

Anatomical and/or - where suitable - perfusion information may be included in the method of the invention for identification of diseases. Anatomical information may for instance be obtained by acquiring a proton or ¹³C-MR image with or without employing a suitable contrast agent before or after the method of the invention.

In another preferred embodiment, the imaging medium comprising hyperpolarised ¹³C-fumarate is administered repeatedly, thus allowing dynamic studies. This is a further advantage of the method according to the invention compared to other MR detection methods using conventional MR contrast agents which - in higher doses - may show toxic effects. Since fumarate seems to be tolerated well, repeated doses of this compound should be possible.

As stated above, the metabolic profile provides information about the metabolic activity of the body, part of the body, cells, tissue, body sample etc. under examination and said information may be used in a subsequent step for, e.g. identifying diseases. However, a physician may also use this information in a further step to choose the appropriate treatment for the patient under examination.

Further, said information may be used to monitor treatment response, e.g. treatment success, of the above mentioned diseases, and its sensitivity makes the method especially suitable for monitoring early treatment response, i.e. response to treatment shortly after its commencement.

In another embodiment, the method of the invention may be used to assess drug efficacy. In said embodiment, potential drugs for curing a certain disease like for instance anti-cancer drugs, may be tested at a very early stage in drug screening, for instance *in vitro* in a cell culture which is a relevant model for said certain disease or in diseased *ex vivo* tissue or a diseased isolated organ. Alternatively, potential drugs for curing a certain disease may be tested at an early stage in drug screening *in vivo*, for instance in an animal model which is relevant for said certain disease. By comparing the metabolic profile of said cell culture, *ex vivo* tissue, isolated or test animal before and after treatment with a potential drug, the efficacy of said drug and thus treatment response and success can be determined which of course provides valuable information in the screening of potential drugs.

Yet another aspect of the invention is a composition comprising TRIS-¹³C₁,₄-fumarate, TRIS-¹³C_{1,4}-fumarate-d₂. ¹³C_{1,4}-fumaric acid or ¹³C_{1,4}-fumaric acid-d₂, a trityl radical and optionally a paramagnetic metal ion.

In a first embodiment, said composition comprises TRIS-¹³C_{1,4}-fumarate or TRIPS-¹³C_{1,4}-fumarate-d₂, a trityl radical and optionally a paramagnetic metal ion. In a preferred embodiment, said trityl radical is a trityl radical of formula (1) wherein M represents hydrogen or sodium and R1 is preferably the same, more preferably a straight chain or branched C₁-C₄-alkyl group, most preferably methyl, ethyl or isopropyl; or R1 is preferably the same, more preferably a straight chain or branched C₁-C₄-alkyl group which is substituted by one hydroxyl group, most preferably - CH₂-CH₂-OH; or R1 is preferably the same and represents -CH₂-OC₂H₄OH. In another preferred embodiment said composition comprises a paramagnetic metal ion, preferably a salt or paramagnetic chelate comprising Gd³⁺ and more a paramagnetic chelate comprising Gd³⁺. Suitably, said composition further comprises a solvent or solvents; preferably an aqueous carrier, e.g. water. Optionally, the composition further comprises a glass former. The TRIS-¹³C_{1,4}-fumarate or TRIS-¹³C_{1,4}-fumarated₂ in said composition may be obtained *in situ* from mixing TRIS with ¹³C_{1,4}-fumaric acid or ¹³C_{1,4}-fumaric acid-d₂ in an aqueous carrier, e.g. water. The aforementioned compositions can be used for obtaining hyperpolarised TRIS-¹³C_{1,4}-fumarate or TRIS-¹³C_{1,4}-fumarate-d₂ by dynamic nuclear polarisation (DNP) with a high polarisation level.

In a second embodiment said composition comprises ¹³C_{1,4}-fumaric acid or ¹³C_{1,4}-fumaric acid-d₂, a trityl radical and optionally a paramagnetic metal ion. In a preferred embodiment, said trityl radical is a trityl radical of formula (1) wherein M represents hydrogen or sodium and R1 is the same or different, preferably the same and preferably represents -CH₂-OCH₃, -CH₂-OC₂H₅, -CH₂-CH₂-OCH₃, -CH₂-SCH₃, -CH₂-SC₂H₅ or -CH₂-CH₂-SCH₃, most preferably -CH₂-CH₂-OCH₃. In another preferred embodiment said composition comprises a paramagnetic metal ion, preferably a salt or paramagnetic chelate comprising Gd³⁺ and more a paramagnetic chelate comprising Gd³⁺. Suitably, said composition further comprises a solvent or solvents; preferably DMSO. Optionally, the composition further comprises a glass former. The aforementioned compositions can be used for obtaining hyperpolarised ¹³C_{1,4}-fumaric acid or hyperpolarised ¹³C_{1,4}-fumaric acid-d₂ by dynamic nuclear polarisation (DNP) with a high polarisation level. Said hyperpolarised ¹³C₁,₄-fumaric acid or hyperpolarised ¹³C_{1,4}-fumaric acid-d₂ can be converted into hyperpolarised ¹³C_{1,4}-fumarate or hyperpolarised ¹³C_{1,4}-fumarate-d₂ by dissolution with a base, e.g. NaOH.

Yet another aspect of the invention is a composition comprising hyperpolarised TRIS-¹³C_{1,4}-fumarate or TRIS-¹³C_{1,4}-fumarate-d₂, hyperpolarised ¹³C_{1,4}-fumaric acid or hyperpolarised ¹³C_{1,4}-fumaric acid-d₂, a trityl radical and optionally a paramagnetic metal ion and/or a glass former, wherein said composition is obtained by dynamic nuclear polarisation.

Yet another aspect of the invention is hyperpolarised TRIS-¹³C_{1,4}-fumarate, hyperpolarised TRIS-¹³C_{1,4}-fumarate-d₂, hyperpolarised ¹³C_{1,4}-fumaric acid or hyperpolarised ¹³C_{1,4}-fumaric acid-d₂. A preferred embodiment of this aspect of the invention is hyperpolarised TRIS-¹³C_{1,4}-fumarate and TRIS-¹³C_{1,4}-fumarate-d₂, preferably hyperpolarised di-TRIS-¹³C_{1,4}-fumarate and di-TRIS-¹³C_{1,4}-fumarate-d₂ which can be used as imaging agent in a composition (imaging medium) for use in a ¹³C-MR detection method.

Yet another aspect of the invention is an imaging medium comprising hyperpolarised TRIS-¹³C_{1,4}-fumarate or hyperpolarised TRIS-¹³C_{1,4}-fumatate-d₂, preferably hyperpolarised di-TRIS-¹³C_{1,4}-fumarate or hyperpolarised di-TRIS-¹³C_{1,4}-fumarated₂.

The imaging medium according to the invention may be used as imaging medium in a method of ¹³C-MR detection.

The imaging medium according to the method of the invention may be used as imaging medium for *in vitro* ¹³C-MR detection, e.g. ¹³C-MR detection of cell cultures, samples, *ex vivo* tissue or isolated organs derived from the human or non-human animal body. For this purpose, the imaging medium is provided as a composition that is suitable for being added to, for instance, cell cultures, samples like urine, blood or saliva, *ex vivo* tissues like biopsy tissues or isolated organs. Such an imaging medium preferably comprises in addition to the imaging agent, i.e. hyperpolarised TRIS-¹³C_{1,4}-fumarate or hyperpolarised TRIS-¹³C_{1,4}-fumarate-d₂, a solvent which is compatible with and used for *in vitro* cell or tissue assays, for instance DMSO or methanol or solvent mixtures comprising an aqueous carrier and a non aqueous solvent, for instance mixtures of DMSO and water or a buffer solution or methanol and water or a buffer solution. As it is apparent for the skilled person, pharmaceutically acceptable carriers, excipients and formulation aids may be present in such an imaging medium but are not required for such a purpose.

Further, the imaging medium according to the method of the invention may be used as imaging medium for *in vivo* ¹³C-MR detection, i.e. ¹³C-MR detection carried out on living human or non-human animal beings. For this purpose, the imaging medium needs to be suitable for administration to a living human or non-human animal body. Hence such an imaging medium preferably comprises in addition to the imaging agent, i.e. TRIS-¹³C_{1,4}-fumarate or hyperpolarised TRIS-¹³C_{1,4}-fumarate-d₂, an aqueous carrier, preferably a physiologically tolerable and pharmaceutically accepted aqueous carrier like water, a buffer solution or saline. It may also comprise DMSO which is a solvent that is used in medicinal applications. Such an imaging medium may further comprise conventional pharmaceutical or veterinary carriers or excipients, e.g. formulation aids such as stabilizers, osmolality adjusting agents, solubilising agents and the like which are conventional for diagnostic compositions in human or veterinary medicine.

Yet another aspect of the invention is a method for producing hyperpolarised ¹³C-fumarate, the method comprising preparing a composition comprising ¹³C-fumaric acid, water, TRIS and optionally a glass former, a DNP agent, preferably a trityl radical and optionally a paramagnetic metal ion carrying out dynamic nuclear polarisation on the composition.

Preferred embodiments of ¹³C-fumaric acid, the trityl radical and the paramagnetic metal ion are described earlier in this application.

### Brief description of the drawings:

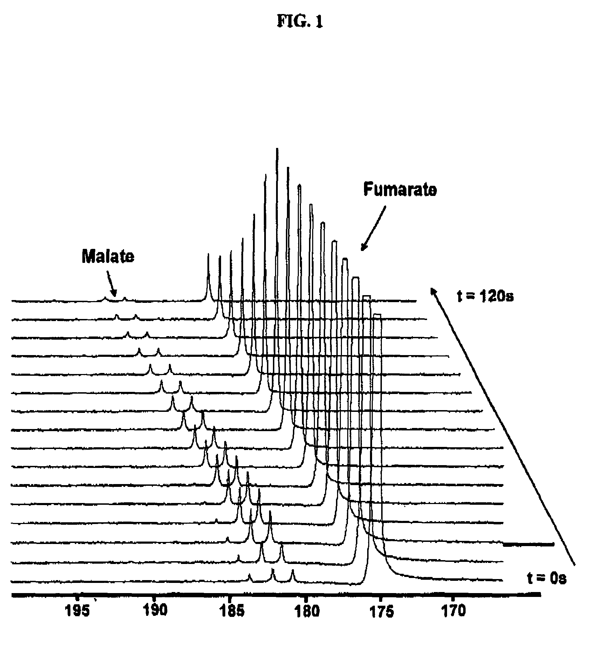

FIG. 1 shows the conversion of ¹³C₁-fumarate-d₂ to ¹³C₁-malate-d₂ and ¹³C₄-malate-d₂ in MDA-MB-231 cells. Time resolved ¹³C-NMR spectra were acquired and the signals of hyperpolarised ¹³C₁-fumarate-d₂ (176.8 ppm), hyperpolarised ¹³C₁-malate-d₂ (182.1 ppm) and hyperpolarised ¹³C₄-malate-d₂ (180.9 ppm) were detected.

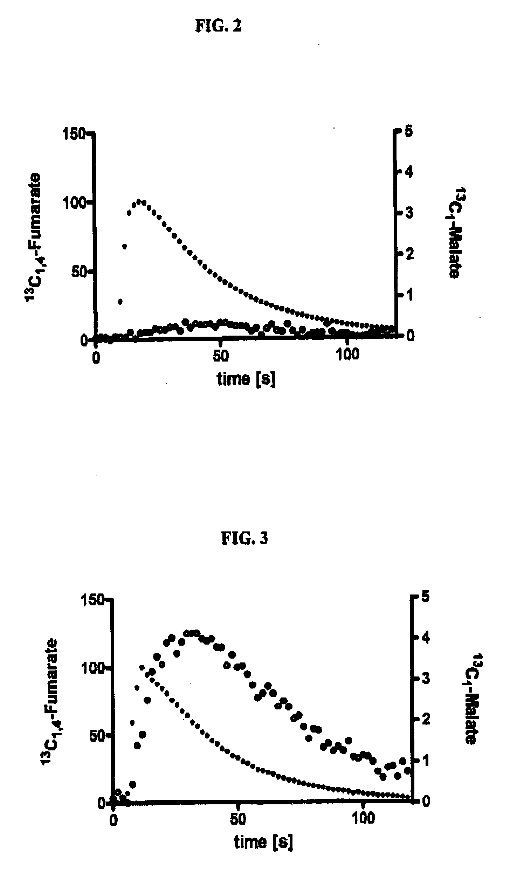

FIG. 2 depicts signal intensities of ¹³C_{1,4}-fumarate and ¹³C_{1,4}-malate over time detected from ¹³C-MR spectroscopy in a mouse hind leg muscle. This is shown as normalised ¹³C_{1,4}-fumarate signal (closed circles) and one of the malate signals (¹³C₁-malate, open circles).
FIG. 3 depicts signal intensities of ¹³C_{1,4}-fumarate and ¹³C_{1,4}malate over time detected from ¹³C-MR spectroscopy in a re-perfused mouse hind leg muscle after 30 min of ischemia. This is shown as normalised ¹³C_{1,4}-fumarate signal (closed circles) and one of the malate signals (¹³C₁-malate, open circles). Compared to Fig. 2, a clear relative increase in malate build-up (higher ¹³C₁-malate signal) can be observed.

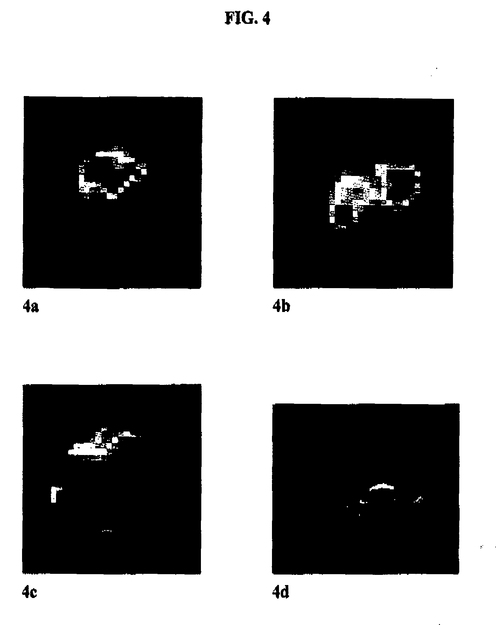

FIG. 4 depicts signal intensities of ¹³C₁-fumarate-d₂, ¹³C₁-malate-d₂ and ¹³C₄-malate-d₂ in a ¹³C-chemical shift image of a lymphoma mouse tumour (EL-4) subcutaneously injected into a mouse flank. The ¹³C-malate signal distribution is shown in FIG. 4a and the ¹³C-fumarate signal distribution is shown in FIG. 4b. The ratio image (¹³C-malate to ¹³C-fumarate) clearly defines the tumour and is shown in FIG. 4c. A ¹H reference was obtained with Omniscan^{™}-enhanced imaging, which is shown in FIG. 4d.

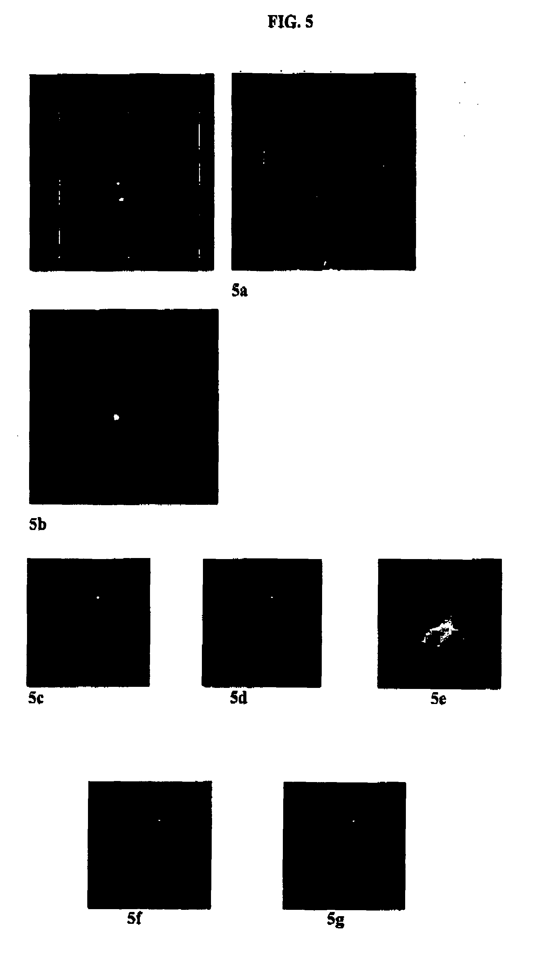

FIG. 5 depicts signal intensities of ¹³C₁-fumarate-d₂, ¹³C₁-malate-d₂ and ¹³C₄-malate-d₂ in a ¹³C-chemical shift image of a normal rat liver. The slice selection is shown in FIGs. Sa, a high resolution proton image is shown as a reference in FIG. 5b. Only ¹³C-fumarate signal can be detected in the normal rat liver, the distribution of which is shown in FIG. 5e. The signals of ¹³C₁-malate and ¹³C₄-malate are too low to detect (FIGs. 5c, 5d) and consequently, the ratio images FIGs 5f, 5g (¹³C₁-malate to ¹³C-fumarate/¹³C₄-malate to ¹³C-fumarate) are empty.

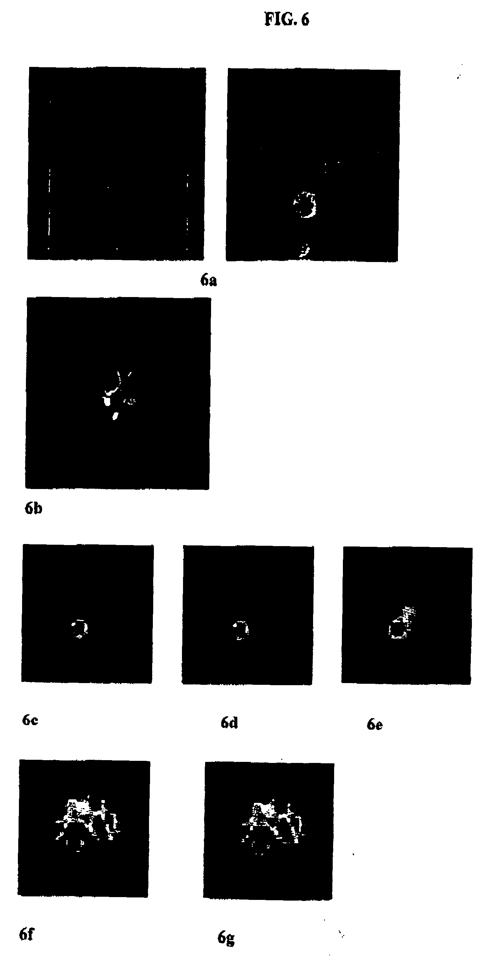

FIG. 6 depicts signal intensities of ¹³C₁-fumamte-d₂, ¹³C₁-malate-d₂ and ¹³C₄-malate-d₂ in a ¹³C-chemical shift image of a fibrotic rat liver. The slice selection is shown in FIGs. 6a, a high resolution proton image is shown as a reference in FIG. 6b. The ¹³C-fumarate distribution is shown in FIG. 6e. The distribution of ¹³C₁-malate and ¹³C₄-malate - which could be detected in the fibrotic liver - is shown in FIGs. 6c, 6d. The ratio images FIG. 6f, 6g (¹³C₁-malate to ¹³C-fumarate/¹³C₄-malate to ¹³C-fumarate) clearly define the diseased liver.

The invention is illustrated by the following non-limiting examples.

### Examples

### Example 1a Production of hyperpolarised TRIS-¹³C_{1,4}-fumarate by the DNP method in the presence of a Gd-chelate as paramagnetic metal ion and a trityl radical as DNP agent

To a micro test tube was added ¹³C_{1,4}-fumaric acid (33.5 mg, 0.284 mmol), TRIS (42 mg, 0.347 mmol) and 22.5 µl water. The test tube was gently heated and sonicated to dissolve all compounds. An aqueous solution of tris(8-carboxy-2,2,6,6-(tetra(hydroxyethyl)-benzo-[1,2-4,5']-bis-(1,3)-dithiole-4-yl)-methyl sodium salt (trityl radical) which had been synthesised according to Example 7 of WO-A1-98/39277 was prepared (140 µmol/g solution) and 6.9 mg of this solution were added to the dissolved TRIS-¹³C_{1,4}-fumarate in the test tube. Further, an aqueous solution of the Gd-chelate of 1,3,5-tris-(N-(DO3A-acetamido)-N-methyl-4-amino-2-methylphenyl)-[1,3,5]triazinane-2,4,6-trione (paramagnetic metal ion) which had been synthesised according to Example 4 of WO-A-2007/064226 was prepared (14.3 µmol/g solution) and 2.8 mg of this solution were added to the test tube with the TRIS-¹³C_{1,4}-fumarate and the trityl radical. The resulting composition was sonicated and gently heated to dissolve all compounds. The composition (80 µl, 12.1 mM in trityl radical and 1.5 mM in Gd³⁺) was transferred with a pipette into a sample cup which was quickly lowered into liquid nitrogen and then inserted into a DNP polariser. The composition was polarised under DNP conditions at 1.2 K in a 3.35 T magnetic field under irradiation with microwave (93.90 GHz). Polarisation was followed by solid state ¹³C-NMR and the solid state polarisation was determined to be 25%.

### Example 1b Production of an imaging medium comprising hyperpolarised ¹³C_{1,4}-fumarate

After 90 minutes of dynamic nuclear polarisation, the obtained frozen polarised composition was dissolved in 6 ml of a solution prepared from 5.87 ml water (100 mg/l EDTA added), 133 µl NaOH aq (2 M) and 30 mg NaCl. The pH of the final solution containing the dissolved composition was 7.2. The ¹³C_{1,4}-fumarate concentration in said final solution was 50 mM.

Liquid state polarisation was determined by liquid state ¹³C-NMR at 400 MHz to be 21 %. T₁ at 9.4 T was 34 s.

### Example 2a Production of hyperpolarised ¹³C_{1,4}-fumaric acid by the DNP method in the presence of a Gd-chelate as paramagnetic metal ion and a trityl radical as DNP agent

A stock solution was prepared by dissolving the trityl radical tris(8-carboxy-2,2,6,6-(tetra(methoxyethyl)-benzo-[1,2-4,5']-bis-(1,3)-dithiole-4-yl)-methyl sodium salt which had been synthesised according to Example 1 of WO-A2-2006/011811 to a final concentration of 18.7 mM and the Gd-chelate of 1,3,5-tris-(N-(DO3A-acetamido)-N-methyl-4-amino-2-methylphenyl)-[1,3,5]triazinane-2,4,6-trione (paramagnetic metal ion) which had been synthesised according to Example 4 of WO-A-2007/064226 to a final Gd³⁺-Concentration of 2.4 mM in DMSO. To a micro test tube was added ¹³C_{1,4}-fumaric acid (39.2 mg, 0.332 mmol) and 60 µl (66.4 mg) of the aforementioned stock solution. The test tube was gently heated and sonicated to dissolve all compounds. The composition was transferred with a pipette into a sample cup which was quickly lowered into liquid nitrogen and then inserted into a DNP polariser. The composition was polarised under DNP conditions at 1.2 K in a 3.35 T magnetic field under irradiation with microwave (93.90 GHz). Polarisation was followed by solid state ¹³C-NMR and the solid state polarisation was determined to be 30 %.

### Example 2b Production of an imaging medium comprising hyperpolarised sodium ¹³C_{1,4}-fumarate

After 90 minutes of dynamic nuclear polarisation, the obtained frozen polarised composition was dissolved in 6 ml of a solution prepared from 5.95 ml water (100 mg/l EDTA added), 57 µl NaOH aq (12 M) and 30 mg NaCl. The pH of the final solution containing the dissolved composition was 6.9. The ¹³C_{1,4}-fumarate concentration in said final solution was 54 mM.

Liquid state polarisation was determined by liquid state ¹³C-NMR at 400 MHz to be 29 %. T₁ at 9.4 T was 34 s.

### Example 3a Production of hyperpolarised ¹³C₁- fumaric acid-4₂ by the DNP method in the presence of a Gd-chelate as paramagnetic metal ion and a trityl radical as DNP agent

To a micro test tube was added ¹³C₁-fumaric acid-d₂ (39.3 mg, 0.33 mmol) and 64 µl (71 mg) of the stock solution of Example 2a. The test tube was gently heated and sonicated to dissolve all compounds. The composition was transferred with a pipette into a sample cup which was quickly lowered into liquid nitrogen and then inserted into a DNP polariser. The composition was polarised under DNP conditions at 1.2 K in a 3.35 T magnetic field under irradiation with microwave (93.90 GHz). Polarisation was followed by solid state ¹³C-NMR and the solid state polarisation was determined to be 41 %.

### Example 3b Production of an imaging medium comprising hyperpolarised sodium ¹³C₁-fumarate-d₂

After 90 minutes of dynamic nuclear polarisation, the obtained frozen polarised composition was dissolved in 6 ml of a solution prepared from 5.95 ml water (100 mg/l EDTA added), 55 µl NaOH aq (12 M) and 30 mg NaCl. The pH of the final solution containing the dissolved composition was 6.9. The ¹³C₁-fumarate-d₂ concentration in said final solution was 54 mM.

Liquid state polarisation was determined by liquid state ¹³C-NMR at 400 MHz to be 40 %. T₁ at 9.4 T was 44 s.

### Example 4 In vitro ¹³C-MR detection using an imaging medium comprising hyperpolarised sodium ¹³C₁-fumarate-d₂

50 µl (2.7 mM in sodium ¹³C₁-fumarate-d₂) of an imaging medium which was prepared according to Example 3 was mixed into 8.10⁶ MDA-MB-231 cells (human epithelial breast cell adenocarcinoma). Signals of ¹³C-fumarate and ¹³C-malate were detected by acquiring a set of ¹³C-MR spectra every 5 s with a 15 degree RF pulse. The result is shown in Fig. 1: ¹³Cmalate was building up over time. The conversion rate was 70 µmol/min·g soluble protein with a peak ¹³C-malate signal after approximately 30 s.

### Example 5 In vivo ¹³C-MR detection with a surface coil placed around a mouse leg using an imaging medium comprising hyperpolarised sodium ¹³C_{1,4}-fumarate

175 µl of an imaging medium comprising hyperpolarised sodium ¹³C_{1,4}-fumarate which was prepared as described in Example 2 was injected into a C57B1/6 mouse over a time period of 6 s. The sodium ¹³C_{1,4}-fumarate concentration in said imaging medium was about 50 mM. A surface coil (tuned for carbon) was positioned around the leg muscle and signals of ¹³C-fumarate and ¹³C-malate were detected by ¹³C-MR spectroscopy which was carried out in a 2.4 T Bruker spectrometer to generate a metabolic profile. A total of 60 ¹³C-spectra were acquired with a repetition time of 2 s and 15 degree RF pulses. The result is shown in Fig. 2. In this Example, the ¹³C-malate signal is approximately 1% of the ¹³C-fumarate signal.

### Example 6 In vivo ¹³C-MR detection with a surface coil placed around a mouse leg after ischemia using an imaging medium comprising hyperpolarised sodium ¹³C_{1,4}-fumarate

Ischemia in a mouse leg muscle was made by strangulation of the hind leg muscle. Following an ischemic period of 30 min the mouse leg muscle was re-perfused for one hour. An imaging medium was injected and ¹³C-MR detection was carried out as described in Example 5. The result is shown in Fig. 3. In this Example, the ¹³C-malate signal is approximately 4% of the ¹³C-fumarate signal. Hence, comparing the metabolic profiles of Examples 4 and 5, a higher ¹³C-malate signal can be seen in the metabolic profile of Example 5 and Example 5 showed an approximate 4 fold relative increase in ¹³C-malate build-up.

### Example 7 In vivo ¹³C-chemical shift imaging with a surface coil placed over a subcutaneous mouse lymphoma (EL-4) using an imaging medium comprising hyperpolarised sodium ¹³C₁-fumarate-d₂

EL-4 cells were injected into a C57B1/6 mouse to generate a subcutaneous mouse lymphoma. 175 µl of an imaging medium comprising hyperpolarised sodium ¹³C₁-fumarate-d₂ which was prepared as described in Example 3 was injected into the mouse over a time period of 6 s. The sodium ¹³C₁-fumarate-d₂ concentration in said imaging medium was about 50 mM, A surface coil (tuned for carbon) was positioned over the subcutaneous tumour and signals of ¹³C-fumarate and ¹³C-malate were detected by ¹³C-MR spectroscopy which was carried out using a 2.4 T Bruker spectrometer to generate a metabolic profile of the tumour and the surrounding healthy tissue. A ¹³C-chemical shift image was acquired with the following parameters: FOV 35x35 mm² x10 mm, matrix size 16x16, 10 degree RF pulse, TR = 35 ms. Total acquisition time was 11 seconds and the chemical shift imaging started 15 seconds after the start of the injection of the imaging medium. Omniscan^{™} (GE Healthcare) - enhanced proton imaging was performed to confirm the tumour position and perfusion. The results are shown in Fig. 4. The ¹³C-fumarate signal is the highest in the large blood vessels; however, the distribution of fumarate is seen over the whole field of view of the surface coil. The ¹³C-malate distribution is confined to the tumour area and a ratio image ¹³C-malate to ¹³C-fumarate) defines the tumour area which is confirmed and shown in the contrast-enhanced proton image.

### Example 8 In vivo ¹³C-chebemical shift imaging of a normal rat liver using an imaging medium comprising hyperpolarised sodium ¹³C₁-fumarate-d₂

2 ml of an imaging medium comprising hyperpolarised sodium ¹³C₁-fumarate-d₂ which was prepared as described in Example 3 was injected into a Wistar rat over a time period of 12 s. The sodium ¹³C₁-fumarate-d₂ concentration in said imaging medium was about 50 mM. A rat coil (tuned for carbon and proton) used and the signal of ¹³C-fumarate was detected by ¹³C-MR spectroscopy which was carried out using a 2.4 T Bruker spectrometer to generate a metabolic profile of the liver region. A ¹³C-chemical shift image was acquired with the following parameters: FOV 55x55 mm² x10 mm, matrix size 16x16, 10 degree RF pulse, TR = 35 ms. Total acquisition time was 11 seconds and the chemical shift imaging started 15 seconds after the start of the injection of the imaging medium. A high resolution proton image was acquired for referencing. The results are shown in Fig 5. Only the ¹³C-fumarate signal could be detected in the normal rat liver.

### Example 9 In vivo ¹³C-chemical shift imaging of a fibrotic rat liver using an imaging medium comprising hyperpolarised sodium ¹³C₁-fumarate-d₂

A Wistar rat had been treated with oil-diluted CCl4 over a time period of 40 days to induce liver fibrosis (Constandinou et al., Methods Mol. Med. 2005, 117, 237-250). 2 ml of an imaging medium comprising hyperpolarised sodium ¹³C₁-fumarate-d₂ which was prepared as described in Example 3 was injected into the rat over a time period of 12 s. The sodium ¹³C₁-fumarate-d₂ concentration in said imaging medium was about 50 mM. A rat coil (tuned for carbon and proton) was used and signals of ¹³C-fumarate and ¹³C-malate were detected by ¹³C-MR spectroscopy which was carried out using a 2.4 T Bruker spectrometer to generate a metabolic profile of the fibrotic liver and surrounding healthy liver tissue. A ¹³C-chemical shift image was acquired with the following parameters: FOV 55x55 mm² x10 mm, matrix size 16x16, 10 degree RF pulse, TR = 35 ms. Total acquisition time was 11 seconds and the chemical shift imaging started 15 seconds after the start of the injection of the imaging medium. A high resolution proton image was acquired for referencing. The results are shown in Fig. 6. Compared to Example 8, both the ¹³C-fumarate and ¹³C-malate signal could be detected in the rat liver. A significantly higher ¹³C-malate signal is seen in the metabolic profile of the fibrotic rat liver compared to the metabolic profile of the normal rat liver.

## Claims

1. Method of ¹³C-MR detection using an imaging medium comprising hyperpolarised ¹³C-fumarate wherein signals of ¹³C-malate and optionally ¹³C-fumarate and/or ¹³C-succinate are detected.

2. The method according to claim 1 wherein signals of ¹³C-malate and ¹³C-fumarate are detected.

3. The method according to claims 1 or 2 wherein said signals are used to generate a metabolic profile.

4. The method according to claim 3, wherein said method is a method of *in vivo* ¹³C-MR detection and said metabolic profile is a metabolic profile of a living human or non-human animal being

5. The method according to claim 3, wherein said method is a method of *in vitro* ¹³C-MR detection and said metabolic profile is a metabolic profile of cells in a cell culture, of samples, of *ex vivo* tissue or of an isolated organ derived from a human or non-human animal being.

6. The method according to claims 1 to 5, wherein said hyperpolarised ¹³C-fumarate is hyperpolarised ¹³C_{1,4}-fumarate, preferably hyperpolarised ¹³C_{1,4}-fumarate-d₂ and more preferably sodium ¹³C_{1,4}-fumarate, TRIS-¹³C_{1,4}-fumarate, sodium ¹³C_{1,4}-fumarate-d₂ or TRIS-¹³C_{1,4}-fumarate-d₂.

7. Composition comprising TRIS-¹³C_{1,4}-fumarate, TRIS-¹³C_{1,4}-fumarate-d₂, ¹³C_{1,4}-fumaric acid or ¹³C_{1,4}-fumaric acid-d₂, a trityl radical and optionally a paramagnetic metal ion.

8. The composition according to claim 7, wherein said paramagnetic metal ion is present and is a paramagnetic chelate comprising Gd³⁺.

9. The composition according to claims 7 and 8, wherein said trityl radical is a trityl radical of formula (1) 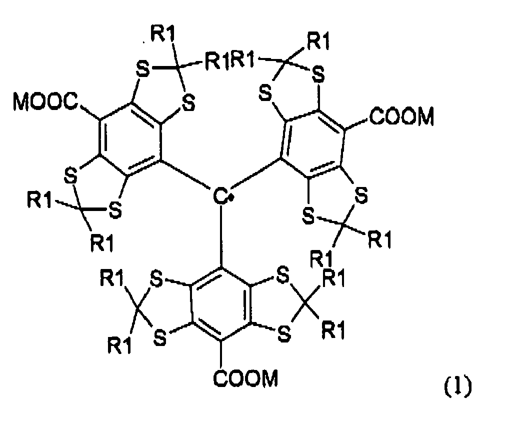 wherein
M represents hydrogen or one equivalent of a cation; and
R1 which is the same or different represents a straight chain or branched C₁-C₆-alkyl group optionally substituted by one or more hydroxyl groups or a group -(CH₂)ₙ-X-R2,
wherein n is 1, 2 or 3;
X is O or S; and
R2 is a straight chain or branched C₁-C₄-alkyl group, optionally substituted by one or more hydroxyl groups.

10. The composition according to claims 7 to 9 for use in dynamic nuclear polarisation.

11. Composition comprising hyperpolarised TRIS-¹³C_{1,4}-fumarate, hyperpolarised TRIS-¹³C_{1,4}-fumarate-d₂, hyperpolarised ¹³C_{1,4}-fumaric acid or hyperpolarised ¹³C_{1,4}-fumaric acid-d₂, a trityl radical and optionally a paramagnetic metal ion, wherein said composition is obtained by dynamic nuclear polarisation of the composition of claims 7 to 10.

12. Imaging medium comprising hyperpolarised TRIS-¹³C_{1,4}-fumarate or hyperpolarised TRIS-¹³C_{1,4}-fumarate-d₂.

13. Imaging medium according to claim 12 for use in the method of claims 1 to 5.

14. Hyperpolarised TRIS-¹³C_{1,4}-fumarate or hyperpolarised TRIS-¹³C_{1,4}-fumarate-d₂
